# EUROPEAN PATENT APPLICATION

(11) **EP 0 679 396 A1**
(43) Date of publication of application: **02.11.1995**
(21) Application number: 95301069.1
(22) Date of filing: 20.02.1995
(51) Int. Cl.: A61K 31/40, A61K 31/405, A61K 31/42, A61K 31/425, A61K 31/415

(54) **Use of 3-substituted-2-oxidole-1-carboxamides for the manufacture of a medicament in the treatment and prevention of ischemia induced myocardial injury and cytokine mediated myocardial injury**

(30) Priority: 02.03.1994 US 204844
(71) Applicant: PFIZER INC., New York, N.Y. 10017 (US)
(72) Inventor: Kitsis, Elizabeth A., Larchmont, New York 10538 (US)
(74) Representative: Wood, David John

(57) **Abstract**

This invention relates to the use of certain 3-substituted 2-oxindole-1-carboxamides and their pharmaceutically acceptable base salts for the manufacture of a medicament for the treatment or prevention of ischemia induced myocardial injury and cytokine mediated myocardial injury in mammals, including humans.

## Description

This invention relates to the use of certain 3-substituted-2-oxindole-1-carboxamides and their pharmaceutically acceptable base salts for the treatment and prevention of ischemia induced myocardial injury and cytokine mediated myocardial injury in mammals, including humans. Examples of such disorders are myocardial stunning and myocardial reperfusion injury.

Tenidap (5-chloro-2,3-dihydro-2-oxo-3-(2-thienylcarbonyl)-indole-1-carboxamide), has the structural formula: 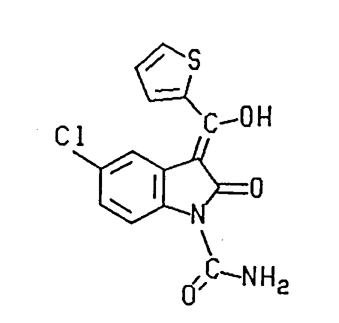 Tenidap and other 3-substituted-2-oxindole-1-carboxamides are referred to in U.S. Patent 4,556,672, which issued on December 3, 1985. This patent refers to the use of such compounds as antiflammatory and analgesic agents, and as inhibitors of both the cyclooxygenase (CO) and lipoxygenase (LO) enzymes. This patent is incorporated herein by reference in its entirety.

Other 3-substituted-2-oxindole derivatives are referred to in United States Patent Application 07/340,113, which was filed on April 18, 1989 and has since been abandoned, and in United States Patent Application 07/712,169, which was filed on May 6, 1991 and allowed on August 18, 1993. Both these applications are incorporated herein by reference in their entirety.

United States Patent 4,861,794, which issued on August 29, 1989, refers to the use of tenidap and certain other 3-substituted-2-oxindole-1-carboxamides to inhibit interleukin-1 biosynthesis in mammals and to treat interleukin-1 mediated disorders and dysfunctions. This patent is incorporated herein by reference in its entirety.

United States Patent 4,853,409, which issued on August 1, 1989, refers to the use of tenidap and certain other 3-substituted-2-oxindole-1-carboxamides to suppress T-cell function in mammals and to treat T-cell mediated autoimmune disorders of the systemic or organ specific type. This patent is incorporated herein by reference in its entirety.

European Patent 277,738, which issued on March 18, 1992, refers to an anhydrous, crystalline form of the sodium salt of tenidap. This patent is incorporated herein by reference in its entirety.

United States Patent 5,008,283, which issued on April 16, 1991, refers to the use of tenidap and its pharmaceutically acceptable base salts to inhibit activation of collagenase, treat collagenase mediated disorders and diseases and inhibit the activity of myeloperoxidase in mammals. This patent is incorporated herein by reference in its entirety.

United States Patent 5,006,547, which issued on April 9, 1991, refers to the use of tenidap and its pharmaceutically acceptable base salts to inhibit the release of elastase by neutrophils in mammals and to treat elastase mediated diseases and dysfunctions in mammals. This patent is incorporated herein by reference in its entirety.

United States Patent 5,122,534, which issued on June 16, 1992, refers to the use of tenidap and its pharmaceutically acceptable base salts to reduce total serum cholesterol, LDL cholesterol and triglycerides in mammals. This patent is incorporated herein by reference in its enirety.

The 2-oxindole-1-carboxamide derivatives referred to below as "compounds of the formula 1", which include tenidap, are also believed to be useful in the treatment and prevention of ischemia induced myocardial injury and cytokine mediated myocardial injury. Examples of such disorders are myocardial stunning and myocardial reperfusion injury.

Myocardial stunning is a transient condition in which severely ischemic myocardium exhibits prolonged impaired contractility after bloodflow has been restored, but in which extensive myocardial necrosis does not occur. Myocardial reperfusion injury is a related condition in which early reperfusion of an ischemic area of myocardium results in extension of myocardial injury; it is characterized by myocyte necrosis. Both of these conditions may be partially mediated by myocardial leukocyte infiltration and activation. (See Ricevuti et al., Atherosclerosis, 91, 1-14 (1991) and Burke et al., Journal of Cardiovascular Pharmacology, 20 (4) 619-629 (1992)). However, there is also evidence that cytokines such as interleukin-1, interleukin-6 and tumor necrosis factor may play a role. (See Finkel, et al., The American Journal of Cardiology, 71, 1231-32 (May 15, 1993); Sturk et al., J. Lab. Clin. Med., 119 (5), 574-579(1992) and Ascer et al., Annals of Vascular Surgery, (1), 69-72 (1992)). Serum levels of interleukin-6 have been reported to be elevated in patients after myocardial infarction, and elevated levels of interleukin-6 have been detected in the pulmonary venous blood of patients following uncomplicated coronary artery bypass surgery. (See Sturk et al., and Finkel et al., referred to above). In addition, interleukin-6 has been demonstrated to act as a negative inotrope on human pectinate and hamster papillary muscles. (See Finkel et al., referred to above).

Administration of the 2-oxindole-1-carboxamide derivatives referred to below, which are both cyclooxygenase inhibitors and cytokine modulators, is expected to reduce myocardial infarct size and improve myocardial contractility.

This invention relates to the use of a compound of formula (I) for the manufacture of a medicament useful for treating or preventing ischemia induced myocardial injury or cytokine mediated myocardial injury in a mammal, including a human, 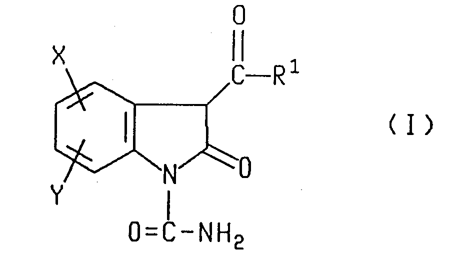 wherein X is selected from the group consisting of hydrogen, fluoro, chloro, bromo, alkyl having 1 to 4 carbons, cycloalkyl having 3 to 7 carbons, alkoxy having 1 to 4 carbons, alkylthio having 1 to 4 carbons, trifluoromethyl, alkylsulfonyl having 1 to 4 carbons, nitro, phenyl, alkanoyl having 2 to 4 carbons, benzoyl, thenoyl, alkanamido having 2 to 4 carbons, benzamido and N,N-dialkylsulfamoyl having 1 to 3 carbons in each of said alkyls;
Y is selected from the group consisting of hydrogen, fluoro, chloro, bromo, alkyl having 1 to 4 carbons, cycloalkyl having 3 to 7 carbons, alkoxy having 1 to 4 carbons, alkylthio having 1 to 4 carbons and trifluoromethyl;
or X and Y, when taken together, are a 4,5-, 5,6- or 6,7-methylenedioxy group or a 4,5-, 5,6- or 6,7-ethylenedioxy group;
or X and Y, when taken together and attached to adjacent carbon atoms, form a divalent radical Z, wherein Z is selected from the group consisting of 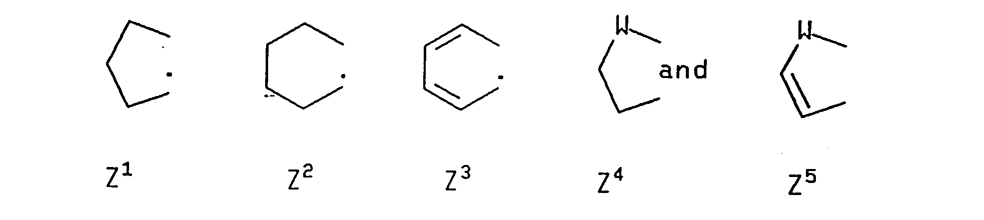 wherein W is oxygen or sulfur;
R¹ is selected from the group consisting of alkyl having 1 to 6 carbons, cycloalkyl having 3 to 7 carbons, cycloalkenyl having 4 to 7 carbons, phenyl, substituted phenyl, phenylalkyl having 1 to 3 carbons in said alkyl, (substituted phenyl)alkyl having 1 to 3 carbons in said alkyl, phenoxyalkyl having 1 to 3 carbons in said alkyl, (substituted phenoxy)alkyl having 1 to 3 carbons in said alkyl, (thiophenoxy)-alkyl having 1 to 3 carbons in said alkyl, naphthyl, bicyclo[2.2.1 ]heptan-2-yl, bicyclo[2.2.1 ]hept-5-en-2-yl and -(CH2),,-Q-RO;
and wherein there are 1 or 2 substituents on said substituted phenyl, said (substituted phenyl)alkyl and said (substituted phenoxy)alkyl, said substituents being independently selected from the group consisting of fluoro, chloro, bromo, alkyl having 1 to 4 carbons, alkoxy having 1 to 4 carbons and trifluoromethyl;
n is zero, 1 or 2;
Q is a divalent radical derived from a compound selected from the group consisting of furan, thiophene, pyrrole, pyrazole, imidazole, thiazole, isothiazole, oxazole, isoxazole, 1,2,3-thiadiazole, 1,3,4-thiadiazole, 1,2,5-thiadiazole, tetrahydrofuran, tetrahydrothiophene, tetrahydropyran, tetrahydrothiopyran, pyridine, pyrimidine, pyrazine, benzo[b]furan and benzo[b]thiophene;
R^{O} is hydrogen, chloro, fluoro, bromo or alkyl having 1 to 3 carbons;

or a pharmaceutically acceptable salt of such compound, or a solvate (e.g., a hemihydrate or monohydrate) of such compound or salt, that is effective in treating or preventing such condition.

Compounds of the formula I, while depicted above in their "keto" tautomeric form, can also exist in their corresponding "enol" tautomeric form. Also, compounds of the formula I may contain chiral centers and therefore may exist in different enantiomeric forms. This invention includes the use of compounds of formula (I) for manufacturing medicaments useful for treating or preventing ischemia induced myocardial injury or cytokine mediated myocardial injury that employ compounds of the formula I in one or both of their tautomeric forms. It also includes such uses that employ compounds of the formula I in their racemic or any of their stereoisomeric forms.

This invention also relates to uses in manufacturing medicaments useful for treating or preventing ischemia induced myocardial injury or cytokine mediated myocardial injury that employ a prodrug of a compound of the formula I. The term "prodrug", as used herein, refers to compounds that are drug precursors which, following administration to and absorption by a mammal, release the drug in vivo via a metabolic process.

A preferred embodiment of this invention relates to the above use wherein the compound used is a compound wherein Y is hydrogen and X is selected from 5-chloro, 6-chloro, 5-fluoro, 6-fluoro, 5-trifluoromethyl and 6-trifluoromethyl, or a pharmaceutically acceptable salt of such compound, or a solvate of such compound or salt.

Another preferred embodiment of this invention relates to the foregoing preferred embodiment wherein, in the compound that is used, R¹ is selected from benzyl, 2-furyl, 2-thienyl, 2-(4-chloro)-thienyl, (2-furyl)-methyl and (2-thienyl)methyl.

Another preferred embodiment of this invention relates to the above use wherein the compound used is a compound wherein X is selected from 5-chloro and 5-fluoro, and Y is selected from 6-chloro and 6-fluoro, or a pharmaceutically acceptable salt of such compound, or a solvate of such compound or salt.

Another preferred embodiment of this invention relates to the foregoing preferred embodiment wherein, in the compound that is used, R¹ is selected from benzyl, 2-furyl, 2-thienyl, 2-(4-chloro)thienyl, (2-furyl)-methyl and (2-thienyl)methyl.

Another preferred embodiment of this invention relates to the above use wherein the compound that is used is selected from the group consisting of:
5-chloro-3-(2-thenoyl)-2-oxindole-1-carboxamide;
5-trifluoromethyl-3-(2-[2-thienyl]acetyl)-2-oxindole-1-carboxamide;
6-fluoro-3-(2-phenylacetyl)-2-oxindole-1-carboxamide;
6-chloro-5-fluoro-3-(2-phenylacetyl)-2-oxindole-1-carboxamide;
5,6-difluoro-3-(2-furoyl)-2-oxindole-1-carboxamide;
5,6-difluoro-3-(2-thenoyl)-2-oxindole-1-carboxamide;
6-chloro-5-fluoro-3-(2-thienyl)-2-oxindole-1-carboxamide;
6-chloro-5-fluoro-3-[2-(3-chloro)thienyl]-2-oxindole-1-carboxamide;
5-chloro-3-[2-(3-chloro)thienyl]-2-oxindole-1-carboxamide;

and the pharmaceutically acceptable salts of the foregoing compounds and the solvates of such compounds and salts.

An especially preferred embodiment of this invention relates to the above use wherein the compound used is tenidap, a pharmaceutically acceptable salt of tenidap, a solvate of tenidap or a solvate of a pharmaceutically acceptable salt of tenidap.

Compounds of the formula I and their pharmaceutically acceptable base salts, solvates and prodrugs may be prepared as described in United States Patent 4,556,672, referred to above.

Compounds of the formula 1 are acidic and therefore form base salts. Such base salts can be formed as described in United States Patent 4,556,672, referred to above. Such salts, within the scope of this invention, include both the organic and inorganic types and include, but are not limited to, the salts formed with ammonia, organic amines, alkali metal hydroxides, alkali metal carbonates, alkali metal bicarbonates, alkali metal hydrides, alkali metal alkoxides, alkaline earth metal hydroxides, alkaline earth metal carbonates, alkaline earth metal hydrides and alkaline earth metal alkoxides. Representative examples of bases that form such base salts include ammonia, primary amines such a n-propylamine, n-butylamine, aniline, cyclohexylamine, benzylamine, p-toluidine, ethanolamine and glucamine; secondary amines such as diethylamine, diethanolamine, N-methylglucamine, N-methylaniline, morpholine, pyrrolidine and piperidine; tertiary amines such as triethylamine, triethanolamine, N,N-dimethylaniline, N-ethylpiperidine and N-methylmorpholine; hydroxides such as sodium hydroxide; alkoxides such as sodium ethoxide and potassium methoxide; hydrides such as calcium hydride and sodium hydride; and carbonates such as potassium carbonate and sodium carbonate. Preferred salts are those of sodium, potassium, ammonium, ethanolamine, diethanolamine and triethanolamine. Particularly preferred are the sodium salts.

An anhydrous crystalline form of such a sodium salt of tenidap is referred to in European Patent 277,738, referred to above.

According to the invention, the medicaments can be manufactured using compounds of formula I and their pharmaceutically acceptable base salts and solvates of such compounds and salts (hereinafter collectively referred to as "the therapeutic agents"). The therapeutic agents can be used to manufacture the medicaments either alone or, preferably, in combination with pharmaceutically acceptable carriers or diluents in a pharmaceutical composition, according to standard pharmaceutical practice. Such medicaments can be administered via a variety of routes, including oral, parenteral, rectal and topical. Parenteral administration, as described herein, includes but is not limited to intravenous, intramuscular, intraperitoneal, subcutaneous, and transdermal administration. It is generally preferred to administer the therapeutic agents orally.

For use in the treatment or prevention of ischemia induced myocardial injury and cytokine mediated myocardial injury, the therapeutic agents are most desirably administered in doses ranging from about 1 mg to about 1.5 gram per day, preferably from about 1 mg to about 300 mg per day, in single or divided doses, although variations will necessarily occur depending upon the weight of the subject being treated, the nature and severity of the subject's condition, the potency of the particular compound being administered and the duration of the treatment.

In some instances, dosage levels below the lower limit of the above dosage ranges may be more than adequate, while in other cases still larger dosages may be employed without causing any harmful or deleterious side effects to occur, provided that such higher dosage levels are first divided into several smaller doses that are to be administered throughout the day.

For purposes of oral administration, tablets containing excipients such as sodium citrate, calcium carbonate and dicalcium phosphate may be employed along with various disintegrants such as starch and preferably potato or tapioca starch, aliginic acid and certain complex silicates, together with binding agents such as polyvinylpyrrolidone, sucrose, gelatin and acacia. Additionally, lubricating agents such as, but not limited to, magnesium stearate, sodium lauryl sulfate and talc are often very useful for tableting purposes. Solid compositions of a similar type may also be employed as fillers in soft elastic and hard filled gelatin capsules. Preferred materials in this connection also include, by way of example and not of limitation, lactose or milk sugar as well as high molecular weight polyethylene glycols. When aqueous suspensions and/or elixirs are desired for oral administration, the essential active ingredient may be combined with various sweetening or flavoring agents, coloring matter or dyes and, if so desired, emulsifying and/or suspending agents, together with diluents such as water, ethanol, propylene glycol, glycerine and various like combinations thereof.

For purposes of parenteral administration, solutions of a therapeutic agent in sesame or peanut oil or in aqueous propylene glycol may be employed, as well as sterile aqueous solutions of the corresponding water soluble base salts previously enumerated. Such aqueous solutions should be suitably buffered if necessary, and the liquid diluent rendered isotonic with sufficient saline or glucose. These particular aqueous solutions are especially suitable for intravenous, intramuscular and subcutaneous injection purposes. In this connection, the sterile aqueous media employed are readily obtained by standard techniques well known to those skilled in the art. For instance, distilled water is ordinarily used as the liquid diluent and the final preparation is passed through a suitable bacterial filter such as a sintered glass filter or a diatomaceous-earth or unglazed porcelain Filter. Preferred filters of this type include the Berkefield, the Chamberland and the Asbestos Disk-Metal Seitz filter, wherein the fluid is sucked into a sterile container with the aid of a suction pump. The necessary steps should be taken throughout the preparation of these injection solutions to insure that the final products are obtained in a sterile condition.

For purposes of transdermal administration, the dosage form of a particular therapeutic agent may include, by way of example, solutions, lotions, ointments, creams, gels, suppositories, rate limiting sustained release formulations and devices. Such dosage forms comprise the particular compound and may include ethanol, water, penetration enhancers and inert carriers such as gel producing materials, mineral oil, emulsifying agents, benzyl alcohol and the like. Specific transdermal flux enhancing compositions are disclosed in European Patent Applications 271,983 and 331,382, which were published, respectively, on June 22 1988 and September 6, 1989. These applications are incorporated herein by reference in thier entireties.

For purposes of topical administration, the dosage form of a particular therapeutic agent may include, by way of example and not of limitation, solutions, lotions, ointments, creams and gels.

## Claims

1. The use of a compound of the formula I, or of a pharmaceutically acceptable salt thereof, or of a solvate of said compound or salt, for the manufacture of a medicament for treating ischemia induced myocardial injury or cytokine mediated myocardial injury in a mammal, 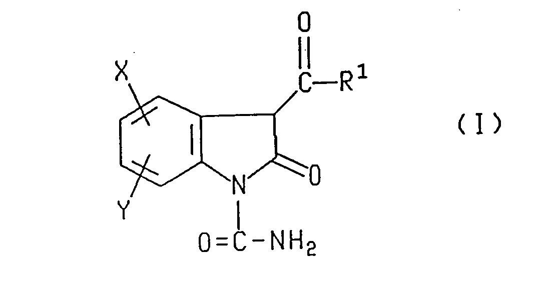 wherein X is selected from the group consisting of hydrogen, fluoro, chloro, bromo, alkyl having 1 to 4 carbons, cycloalkyl having 3 to 7 carbons, alkoxy having 1 to 4 carbons, alkylthio having 1 to 4 carbons, trifluoromethyl, alkylsulfonyl having 1 to 4 carbons, nitro, phenyl, alkanoyl having 2 to 4 carbons, benzoyl, thenoyl, alkanamido having 2 to 4 carbons, benzamido and N,N-dialkylsulfamoyl having 1 to 3 carbons in each of said alkyls;
Y is selected from the group consisting of hydrogen, fluoro, chloro, bromo, alkyl having 1 to 4 carbons, cycloalkyl having 3 to 7 carbons, alkoxy having 1 to 4 carbons, alkylthio having 1 to 4 carbons and trifluoromethyl;
or X and Y, when taken together, are a 4,5-, 5,6- or 6,7-methylenedioxy group or a 4,5-, 5,6- or 6,7-ethylenedioxy group;
or X and Y, when taken together and attached to adjacent carbon atoms, form a divalent radical Z, wherein Z is selected from the group consisting of 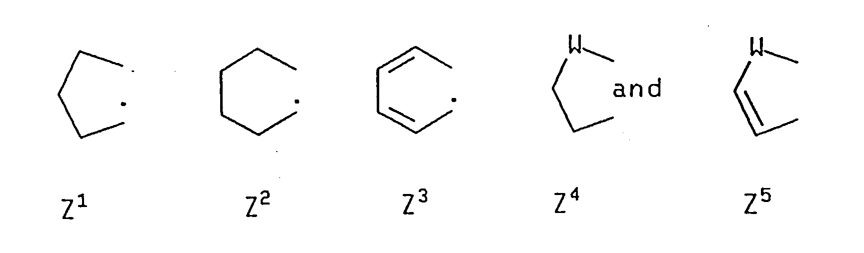 wherein W is oxygen or sulfur;
R¹ is selected from the group consisting of alkyl having 1 to 6 carbons, cycloalkyl having 3 to 7 carbons, cycloalkyl having 4 to 7 carbons, phenyl, substituted phenyl, phenylalkyl having 1 to 3 carbons in said alkyl, (substituted phenyl)alkyl having 1 to 3 carbons in said alkyl, phenoxyalkyl having 1 to 3 carbons in said alkyl, (substituted phenoxy)alkyl having 1 to 3 carbons in said alkyl, (thiophenoxy)-alkyl havbing 1 to 3 carbons in said alkyl, naphthyl, bicyclo[2.2.1 ]heptan-2-yl, bicyclo-[2.2.1]hept-5-en-2-yl and -(CH₂)n-Q-R^{O};
and wherein there are 1 or 2 substituents on said substituted phenyl, said (substituted phenyl)alkyl and said (substituted phenoxy)alkyl, said substituents being independently selected from the group consisting of fluoro, chloro, bromo, alkyl having 1 to 4 carbons, alkoxy having 1 to 4 carbons and trifluoromethyl;
n is zero, 1 or 2;
Q is a divalent radical derived from a compound selected from the group consisting of furan, thiophene, pyrrole, pyrazole, imidazole, thiazole, isothiazole, oxazole, isoxazole, 1,2,3-thiadiazole, 1,3,4-thiadiazole, 1,2,5-thiadiazole, tetrahydrofuran, tetrahydrothiophene, tetrahydropyran, tetrahydrothiopyran, pyridine, pyrimidine, pyrazine, benzo[b]furan and benzo[b]thiophene;
R^{O} is hydrogen, chloro, fluoro, bromo or alkyl having 1 to 3 carbons.

2. The use according to claim 1, wherein the compound is one wherein Y is hydrogen and X is selected from 5-chloro, 6-chloro, 5-fluoro, 6-fluoro, 5-trifluoromethyl and 6-trifluoromethyl.

3. The use according to claim 2, wherein the compound is one where R¹ is selected from benzyl, 2-furyl, 2-thienyl, (2-furyl)methyl and (2-thienyl)methyl.

4. The use according to claim 1, wherein the compound is one wherein X is selected from 5-chloro and 5-fluoro, and Y is selected from 6-chloro and 6-fluoro.

5. The use according to claim 4, wherein the compound is one wherein R¹ is selected from benzyl, 2-furyl, 2-thienyl, (2-furyl)methyl and (2-thienyl)methyl.

6. The use according to claim 1, wherein the compound is selected from the group consisting of:
5-chloro-3-(2-thenoyl)-2-oxindole 1-carboxamide;
5-trifluoromethyl-3-(2-[2-thienyl]acetyl)-2-oxindole-1-carboxamide;
6-fluoro-3-(2-phenylacetyl)-2-oxindole-1-carboxamide;
6-chloro-5-fluoro-3-(2-phenylacetyl)-2-oxindole-1-carboxamide;
5,6-difluoro-3-(2-furoyl)-2-oxindole-1-carboxamide;
5,6-difluoro-3-(2-thenoyl)-2-oxindole-1-carboxamide;
6-chloro-5-fluoro-3-(2-thienyl)-2-oxindole-1-carboxamide;
6-chloro-5-fluoro-3-[2-(3-chloro)thienyl]-2-oxindole-1-carboxamide;
5-chloro-3-[2-(3-chloro)thienyl]-2-oxindole-1-carboxamide;
and the pharmaceutically acceptable salts of the foregoing compounds and solvates of such compounds and salts.

7. The use according to claim 1 wherein the compound is tenidap, a pharmaceutically acceptable salt of tenidap, a solvate of tenidap or a solvate of a pharmaceutically acceptable salt of tenidap.

8. The use according to claim 7 wherein the sodium salt of tenidap is used.

9. The use according to claim 7 wherein tenidap, a pharmaceutically acceptable base salt of tenidap, a solvate of tenidap or a solvate of a pharmaceutically acceptable base salt of tenidap is used to manufacture a medicament for oral administration.

10. The use according to claim 9 wherein the sodium salt of tenidap is used.

11. The use according to claim 7 wherein tenidap, a pharmaceutically acceptable base salt of tenidap, a solvate of tenidap or a solvate of a pharmaceutically acceptable base salt of tenidap is used to manufacture a medicament for parenteral administration.

12. The use according to claim 11 wherein the sodium salt of tenidap is used.
